# EUROPEAN PATENT SPECIFICATION

(11) **EP 3 268 352 B1**
(45) Date of publication and mention of the grant of the patent: **10.06.2020**
(21) Application number: 16714250.4
(22) Date of filing: 14.03.2016
(51) Int. Cl.: C07C 381/00, C08J 3/24

(54) **NON EXPLOSIVE BIS-SULFONYL AZIDES**
NICHTEXPLOSIVE BIS-SULFONYL-AZIDE
BIS-SULFONYL AZIDES NON EXPLOSIFS

(30) Priority: 13.03.2015 WO PCT/EP2015/055345
(43) Date of publication of application: 17.01.2018
(73) Proprietor: Dynamit Nobel GmbH Explosivstoff- und Systemtechnik, 51377 Leverkusen (DE)
(72) Inventor: HALLER, Jan, 51379 Leverkusen (DE); DURAND, Thierry, 69008 Lyon (FR)
(74) Representative: August Debouzy
(86) International application number: PCT/EP2016/055480
(87) International publication number: WO 2016/146597

(56) References cited:
- DE-A1- 3 404 456
- GB-A- 1 129 399
- US-A- 3 298 975
- US-A- 3 652 599
- US-A1- 2011 178 243
- R.J. CREMLYN, ET AL.: "Sulfonyl derivatives of succindianilide and glutardianilide", JOURNAL OF THE CHEMICAL SOCIETY OF PAKISTAN, vol. 8, no. 4, December 1986 (1986-12), pages 491-496, XP055224097, Chemical Soceity of Pakistan, Karachi, PK ISSN: 0253-5106
- R.J. CREMLYN, ET AL.: "Chlorosulfonation of some anilides", JOURNAL OF THE CHEMICAL SOCIETY OF PAKISTAN, vol. 7, no. 2, June 1985 (1985-06), pages 111-124, XP055224102, Chemical Soceity of Pakistan, Karachi, PK ISSN: 0253-5106
- H. ULRICH, ET AL.: "New syntheses of functional arenesulfonyl azides", JOURNAL OF ORGANIC CHEMISTRY, vol. 40, no. 6, March 1975 (1975-03), pages 802-804, XP055224107, American Chemical Society, Washington, DC, US ISSN: 0022-3263, DOI: 10.1021/jo00894a032

## Description

### FIELD OF THE INVENTION

The present invention relates to a class of sulfonyl azide compounds used as cross-linking agents and having non explosive properties.

### TECHNICAL BACKGROUND

Chemical cross-linkers such as organic peroxides and silanes are able to link polymer chains, so as to create three-dimensional polymer networks. Cross-linking reactions lead to superior material characteristics of the polymers compared to the corresponding non-cross-linked raw polymers.

In particular, thermoplastic polyolefins and elastomers are polymers which are frequently modified by cross-linking in order to improve their rheological properties such as melt strength and their general processability and/or their final mechanical properties. The physical properties of the cross-linked polymers, for example specific molecular weight distribution, tensile and tear strength, and snappiness are generally improved.

Peroxide-based cross-linking processes are radical processes which are difficult to control and generate decomposition byproducts. Silane-based processes are two-step processes also initiated by a radical peroxide reaction to graft a silane to a polymer. The second step is then the cross-linking reaction which involves the hydrolysis of alkoxy groups.

Azide compounds, and more particularly bis sulfonyl azide compounds, are known as advantageous cross-linkers in the art.

A sulfonylazide coupling process relies on the thermal decomposition of the sulfonylazide, which generates reactive nitrenes that in the singlet state are known to undergo carbon-hydrogen (C-H) insertion to form sulfonylamido linkages. This process can be controlled by process temperature and concentration of the azide. The main advantages of sulfonylazide cross-linking versus radical cross-linking are a better processability (control of the reaction, no adverse effect on flow rate), less byproducts and a more homogeneous cross-linking of the polymers (resulting in better mechanical properties).

Document US 2011/178243 describes a process for attaching a modifying agent, e.g., a coupling agent such as a poly(sulfonyl) azide, to a substrate, e.g., a polyolefin.

For example, document GB 1129399 describes the production of polymer fibers with the use of cross-linkers, such as bis azides. Sulfonyl azides and azido formates are mentioned, in particular 1,10-decane bis(sulfonazide) and 4,4'-diphenylether bis(sulfonazide).

Document US 3,298,975 relates to partially foamed polypropylene compositions including sulfonyl azides as cross-linkers. A novel synthesis of a chloroaliphatic poly(sulfonyl azide) is described, but non-chlorinated alkane bis(sulfonyl azides) are also mentioned, such as 1,6-bis(4-azidosulfonyl phenyl)hexane.

Documents US 3,352,798 and GB 1080619 relate to the use of poly(sulfonyl azides) as cross-linkers for a broad range of polymers. Aralkylene and some sulfonyl azide compounds including ether groups are mentioned.

Documents BE 638643, BE 638644 and BE 622066 relate to the production of polypropylene foams or hollow polypropylene plastic, using sulfonyl azide or azidoformate cross-linkers similar to the ones mentioned in the other documents above.

One important disadvantage of most of the above chemical sulfonylazide cross-linking systems is that the cross-linkers are explosive. Therefore, they have to be used with phlegmatizing agents, which makes the cross-linking process more expensive. In addition, phlegmatizing agents are not easy to handle, require precise industrial adjustments, and are not desirable in every application.

For instance, DPO-BSA (4,4'-diphenylether-bis(sulfonazide)) is a well-known bis-sulfonyl azide cross-linker currently used in the industry for polymer manufacturing or post-treatment. But it has the disadvantage of being explosive according to two tests of the UN-manual for tests and criteria and the (German) explosives act (shock sensitive and explosive at heating under partial confinement in the Koenen-test). This compound needs to be phlegmatized with an inert non-explosive substance (phlegmatizing agent) for transportation, safe handling and technical use, for example in extrusion processes. A typical phlegmatized composition contains less than 67%(w/w) DPO-BSA and more than 33% (w/w) stabilizer, such as Irganox® 1010. Phlegmatizing agents have to be adjusted to the plastics manufacturing process, requiring development and testing of every new combination.

There is therefore a need for non or less explosive cross-linkers, with no need or less need for phlegmatization, such cross-linkers being advantageously synthesized in an easy way. Non or less explosive cross-linkers would also result in reduced costs of synthesis, formulation and transportation.

### SUMMARY

It is a first object of the invention to provide a compound according to claim 1. The present patent application also discloses a compound of the general formula

(I) N₃O₂S-Ar-L-Ar'-SO₂N₃,

wherein:
- Ar and Ar' are aromatic building blocks; and
- L is a linking group comprising at least one heteroatom as well as an organic chain denoted as Sp" and having at least two carbon atoms.
   not including:

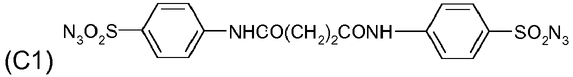

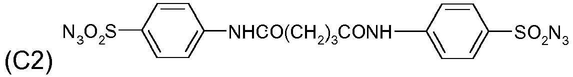

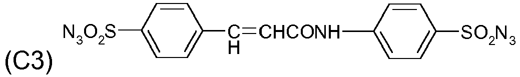

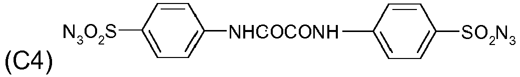

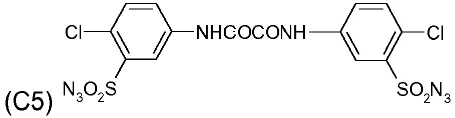

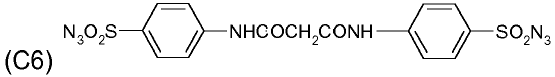

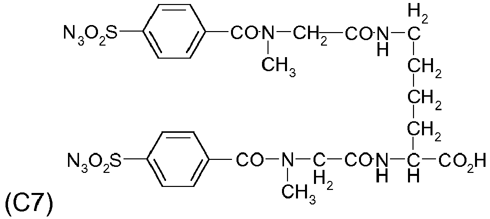

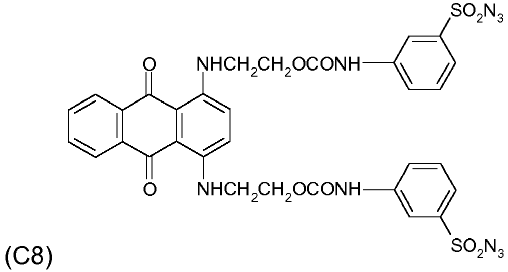

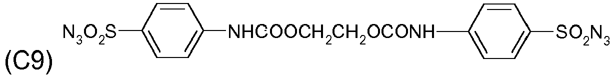

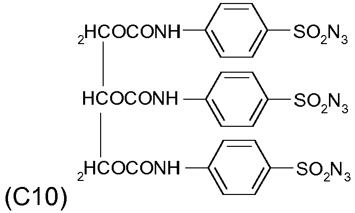

According to one disclosure, compounds of the general formula:

(III) N₃O₂S-(Ar-z)-R₁-Sp"'-(R₂-(Ar-z)-SO₂N₃)ₓ,

wherein:
- Ar is an aromatic building block;
- Z is a hydrogen atom or a Chlorine atom
- x is superior or equal to 1
- R₁ is a linking group such as -CO-NH- or -NH-CO- or -CON-CH₃
- R₂ is a linking group such as -CO-NH- or -NH-CO- or -CON-CH₃ or -CH=CH-
- Sp'" is an organic chain with at least one carbon atom or a covalent bond.
are not included.

According to one disclosure, the at least one heteroatom in the linking group L is selected from sulfur, nitrogen, and oxygen.

According to one disclosure, the organic chain Sp" is an aliphatic chain, and is preferably linear.

According to one disclosure, the organic chain Sp" comprises from 2 to 20 carbon atoms, preferably from 6 to 16 carbon atoms.

According to one disclosure, Ar is a phenyl group, or Ar' is a phenyl group, and preferably Ar and Ar' are both phenyl groups, said phenyl groups being substituted or not.

According to one disclosure, L is Y-Sp"-Y', wherein:
- Y and Y' are both functional groups comprising a heteroatom, which are preferably independently selected from -O-, -NH-, -NR-, -OC(=O)NH-, -OC(=O)NR'-, -OC(=O)O-, -NHC(=O)NH-, -NHC(=O)O-, -NHC(=O)NR'-, -NRC(=O)NH-, -NRC(=O)O-, -NRC(=O)NR'-, -S-, -SO₂-, -C(=O)O-, -C(=O)NH-, -C(=O)NR-, -C(=O)-, -OC(=O)-, -NHC(=O)- and -NRC(=O)-, R and R' being alkyl or aryl groups; and
- Sp" is as defined above.

According to one disclosure, Ar and Ar' are phenyl groups, substituted or not; L is O-Sp"-O; and the organic chain Sp" is an aliphatic chain which preferably comprises at least 3, preferably at least 4, more preferably at least 5, and most preferably at least 6 carbon atoms.

According to one disclosure, the compound has the general formula:

(I") (N₃O₂S-Ar-Y)₂Sp",

wherein Ar, Y and Sp" are as defined above.

According to one disclosure, the compound is 1,6-bis-(4-azidosulfonylphenoxy)-hexane.

According to one disclosure, the compound is 1,10-bis-(4-azidosulfonylphenoxy)-decane.

According to one disclosure, the compound is 1,2-bis-(4-azidosulfonylphenoxy)-hexadecane.

According to one disclosure, the compound is an isomeric mixture of 4,8-bis-(4-azidosulfonylphenoxymethyl)-tricyclo[5.2.1.0^{2.6}]decane.

According to one disclosure, the compound is 1,4-bis-(4-azidosulfonylphenoxymethyl)-cyclohexane.

According to one disclosure, the compound is 1,n-bis-(4-azidosulfonylphenyl)-polyethylene glycol ether.

It is a second object of the invention to provide a compound according to claim 4. The present patent application also discloses a compound of the general formula 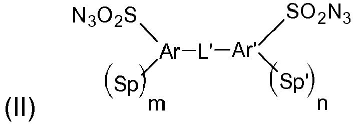 wherein:
- Ar and Ar' are aromatic building blocks;
- L' is a covalent bond or a linking group;
- m and n are both 0 or 1, at least one of m and n being 1;
- Sp and Sp' are organic chains having at least two carbon atoms. not including (C11) :

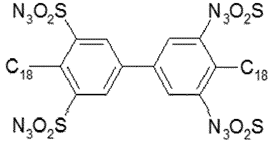

According to one disclosure, L' comprises at least one heteroatom, preferably selected from sulfur, nitrogen, and oxygen.

According to one disclosure, m is 1 and n is 0; or m is 0 and n is 1.

According to one disclosure, Sp and/or Sp' is an aliphatic chain, and is preferably linear.

According to one disclosure, Sp and/or Sp' comprises from 2 to 20 carbon atoms, preferably from 6 to 18 carbon atoms.

According to one disclosure, L' is selected from -O-, -NH-, -NR-, - OC(=O)NH-, -OC(=O)NR'-, -OC(=O)O-, -NHC(=O)NH-, -NHC(=O)O-, -NHC(=O)NR'-, -NRC(=O)NH-, -NRC(=O)O-, -NRC(=O)NR'-, -S-, -SO₂-, -C(=O)O-, -C(=O)NH-, -C(=O)NR-, -C(=O)-, -OC(=O)-, -NHC(=O)- and -NRC(=O)-, R and R' being alkyl or aryl groups.

According to one disclosure, L' is -O-, Ar and Ar' are phenyl groups, and Sp and/or Sp' is an aliphatic chain comprising at least 3, preferably at least 4, more preferably at least 5 and most preferably at least 6 carbon atoms.

According to one disclosure, the compound is 4,4'-bis-(azidosulfonyl)-3-pentadecyl diphenylether.

It is another object of the disclosure to provide a process for producing any of the abovementioned compounds, which comprises providing starting compounds A and B, wherein A and B are substituted aromatic compounds, and which comprises a step of reacting A and B.

According to one embodiment, this process is for producing a compound according to the first object of the invention, wherein the starting compounds A and B are reacted in the presence of an additional compound Z-Sp"-Z', Sp" being the organic chain defined above, and Z and Z' being two reactive groups ; and wherein A and B are preferably identical.

According to one embodiment, this process is for producing a compound according to the second object of the invention, wherein the step of reacting A and B is followed by a step of chlorosulfonating the obtained product, and then a step of reacting the obtained product with sodium azide.

It is another object of the invention to provide a composition comprising the compound according to any one of claims 1 to 6, and containing at most 50% (w/w), preferably at most 40% (w/w), more preferably at most 30% (w/w), even more preferably at most 20% (w/w), most preferably at most 10% (w/w) of a phlegmatizing agent.

It is another object of the invention to provide the use according to claim 8. The present patent application also discloses the use of the compound of the general formula

(I) N₃O2S-Ar-L-Ar'-SO2N₃,

wherein:
- Ar and Ar' are aromatic building blocks; and
   L is a linking group comprising at least one heteroatom as well as an organic chain denoted as Sp" and having at least two carbon atoms.
or of the general formula: 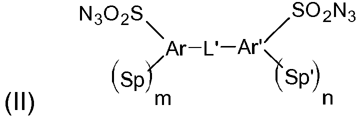 wherein:
- Ar and Ar' are aromatic building blocks;
- L' is a covalent bond or a linking group;
- m and n are both 0 or 1, at least one of m and n being 1;
Sp and Sp' are organic chains having at least two carbon atoms as a cross-linker.

According to one embodiment, the above use is preferably with the compounds according to the above mentioned aspect as a cross-linker.

According to one embodiment, said compound is used with at most 50% (w/w), preferably at most 40% (w/w), more preferably at most 30% (w/w), even more preferably at most 20% (w/w), most preferably at most 10% (w/w) of a phlegmatizing agent.

According to one embodiment, said compound is used without any phlegmatizing agent.

According to one embodiment, this use is a use as a cross-linker in the manufacture of polymers.

According to one embodiment, this use is a use as a cross-linker in the manufacture of polyolefins, polyolefin copolymers and blends of polyolefins, preferably in the manufacture of polypropylene or polyethylene.

According to one embodiment, this use is a use as a cross-linker in the manufacture of elastomers, elastomer copolymers and blends of elastomers, preferably in the manufacture of EPM, EPDM and EPM-polyolefin blends.

The present invention overcomes the disadvantages of prior art. It more particularly provides new cross-linking agents that are not explosive or are less explosive than prior art compounds, and thus do not require a phlegmatizing agent, or at least require smaller amounts of phlegmatizing agent.

Without wishing to be bound by any theory, the inventors believe that this is accomplished thanks to the integration of additional carbon atoms in a spacer or side chain in a bis(aryl sulfonyl azide) molecule. The increase in molecular weight achieved owing to the spacer or side chain is believed to result in reduced or suppressed explosiveness. Furthermore, reduced crystallinity is believed to be present in the compounds of the invention, also resulting in reduced or suppressed explosiveness as no large crystals are formed in the manufacturing process.

In particular, the compounds of the present invention have reduced explosiveness relative to DPO-BSA, which is one important sulfonylazide cross-linker currently used in the industry.

The spacer can be positioned between the two aryl sulfonyl azide groups, or it can be positioned as a substituent or side chain on one or both of the aryl sulfonyl azide groups.

Furthermore, the compounds of the invention are relatively easy to synthesize, in particular when one or more heteroatoms are present in the linker between the two aryl sulfonyl azide groups. This stands in contrast with compounds mentioned in the prior art such as 1,6-bis(4-azidosulfonylphenyl)hexane, which are believed to be more difficult to synthesize.

### BRIEF DESCRIPTION OF THE FIGURES

Figure 1 shows the evolution of complex viscosity (Y-axis, in Pa.s) over the frequency (X-axis, in rad/s) of POHOP-SA in HDPE.
Figure 2 shows the evolution of storage and loss modulus (Y-axis, in Pa) over the frequency (X-axis, in rad/s) for various molarity of POHOP-SA in HDPE; more particularly 0.329 mmol/kg and 0.657 mmol/kg of POHOP-SA in HDPE.
Figure 3 shows the evolution of storage and loss modulus (Y-axis, in Pa) over the frequency (X-axis, in rad/s) for 2.629 mmol/kg of POHOP-SA in HDPE.
Figure 4 shows the evolution of storage and loss modulus (Y-axis, in Pa) over the frequency (X-axis, in rad/s) for 2.629 mmol/kg of POHOP-SA in HDPE.
Figure 5 shows the evolution of storage and loss modulus (Y-axis, in Pa) over the frequency (X-axis, in rad/s) for various molarity of PODOP-SA in HDPE; more particularly 0.329 mmol/kg and 0.657 mmol/kg of POHOP-SA in HDPE.
Figure 6 shows the evolution of storage and loss modulus (Y-axis, in Pa) over the frequency (X-axis, in rad/s) for 2.629 mmol/kg of PODOP-SA in HDPE.
Figure 7 shows the evolution of complex viscosity (Y-axis, in Pa.s) over the frequency (X-axis, in rad/s) of PODOP-SA and DPO-BSA.
Figure 8 shows the evolution of storage and loss modulus (Y-axis, in Pa) over the frequency (X-axis, in rad/s) for 2.629 mmol/kg of PODOP-SA and 2.629 mmol/kg of DPO-BSA.

### DETAILED DESCRIPTION

The invention will now be described in more detail without limitation in the following description.

The compounds of the invention fall into two categories, namely type 1 and type 2.

Compounds of the invention of type 1 have following general formula:

(I) N₃O₂S-Ar-L-Ar'-SO₂N₃.

Compounds of the invention of type 2 have following general formula:

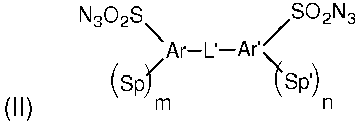

In the above formulas, Ar and Ar' are aromatic building blocks.

The aromatic building blocks Ar and Ar' are aryl moieties, *i.e.* they are or they comprise respective aromatic rings. These aromatic rings can be substituted or not. In case substituents are present, they can in particular be similar to the side chains Sp and Sp' which are described in more detail below. In this case, the aromatic ring of the Ar / Ar' aromatic building block can be linked to more than one side chain Sp / Sp', for instance to two side chains Sp / Sp', or to three side chains Sp / Sp', or to four side chains Sp / Sp', including the one shown in formula (II). The various side chains Sp / Sp' linked to the aromatic ring can be identical or different.

Ar and Ar' can be identical or different, preferably identical. Ar and Ar' are phenyl groups.

In formula (I), L is a linking group or linker.

The linking group L comprises at least one heteroatom and a spacer denoted as Sp".

The heteroatom is oxygen.

In formula (II), L' is a linking group; m and n are each independently 0 or 1; and Sp and Sp' are side chains - which can also be called spacers in the context of this application.

When m=0, Sp is not present, whereas when m=1, Sp is present.

When n=0, Sp' is not present, whereas when n=1, Sp' is present.

At least one of Sp and Sp' is present, which means that at least one of m and n is 1. According to the invention, only one of Sp and Sp' is present.

In both of the above formulas, Sp, Sp' and Sp" are organic chains. In particular each of Sp, Sp' and Sp" can be an aromatic chain or an aromatic group-containing chain. However, preferably, each of Sp, Sp' and Sp" is an aliphatic chain. Each of Sp, Sp' and Sp" comprises at least two carbon atoms.

According to one embodiment, the chain Sp or Sp' or Sp" has 2 carbon atoms.

According to one embodiment, the chain Sp or Sp' or Sp" has 3 carbon atoms.

According to one embodiment, the chain Sp or Sp' or Sp" has 4 carbon atoms.

According to one embodiment, the chain Sp or Sp' or Sp" has 5 carbon atoms.

According to one embodiment, the chain Sp or Sp' or Sp" has 6 carbon atoms.

According to one embodiment, the chain Sp or Sp' or Sp" has 7 carbon atoms.

According to one embodiment, the chain Sp or Sp' or Sp" has 8 carbon atoms.

According to one embodiment, the chain Sp or Sp' or Sp" has 9 carbon atoms.

According to one embodiment, the chain Sp or Sp' or Sp" has 10 carbon atoms.

According to one embodiment, the chain Sp or Sp' or Sp" has 11 carbon atoms.

According to one embodiment, the chain Sp or Sp' or Sp" has 12 carbon atoms.

According to one embodiment, the chain Sp or Sp' or Sp" has 13 carbon atoms.

According to one embodiment, the chain Sp or Sp' or Sp" has 14 carbon atoms.

According to one embodiment, the chain Sp or Sp' or Sp" has 15 carbon atoms.

According to one embodiment, the chain Sp or Sp' or Sp" has 16 carbon atoms.

According to one embodiment, the chain Sp or Sp' or Sp" has 17 carbon atoms.

According to one embodiment, the chain Sp or Sp' or Sp" has 18 carbon atoms.

According to one embodiment, the chain Sp or Sp' or Sp" has 19 carbon atoms.

According to one embodiment, the chain Sp or Sp' or Sp" has 20 carbon atoms.

According to one embodiment, the chain Sp or Sp' or Sp" has more than 20 carbon atoms.

The chain Sp or Sp' or Sp" may be linear, branched or cyclic. It can be saturated or unsaturated and is most preferably saturated. It can be substituted or, more preferably, non-substituted. In case it is substituted, alkyl chain substituents are preferred, as they contribute to a reduced tendency to explosiveness.

The compounds of the invention can be both of type 1 and type 2 at the same time. Such compounds can be compounds of formula (I), wherein at least one of Ar and Ar' comprises an aromatic ring with a respective chain Sp or Sp' as defined above linked to said aromatic ring. Such compounds can also be compounds of formula (II), wherein L' is a linking group similar to a linking group L of formula (I) defined above.

Preferred compounds of type 1 are of the general formula:

(I') N₃O₂S-Ar-Y-Sp"-Y'-Ar'-SO₂N₃.

In this formula, Sp" is as defined above; Y and Y' are two functional groups which are identical. The two groups N₃O₂S-Ar-Y- and N₃O₂S-Ar'-Y'-respectively linked to the spacer Sp" are identical. Such compounds are of the general formula:

(I") (N₃O₂S-Ar-Y)₂Sp".

The functional groups Y and Y' comprise at least one heteroatom, selected from oxygen.

They are selected from the following groups: -O-, - -C(=O)O-, - OC(=O)-.

Preferred examples of compounds of type 1 are:
- 1,6-bis-(4-azidosulfonylphenoxy)-hexane, also designated POHOP-SA, having the following formula: 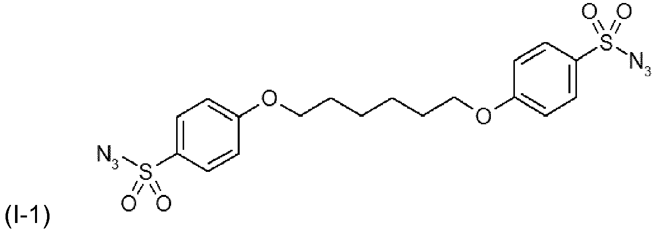
- 1,10-bis-(4-azidosulfonylphenoxy)-decane, also designated PODOP-SA, having the following formula: 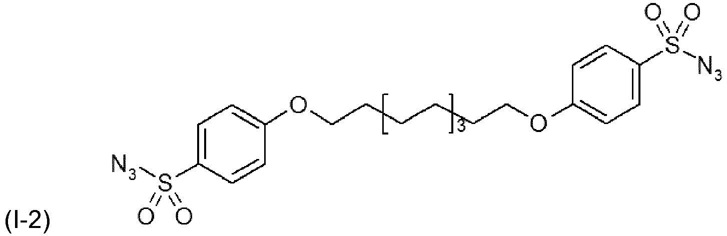
- 1,2-bis-(4-azidosulfonylphenoxy)-hexadecane, also designated POTDEOP-SA, having the following formula: 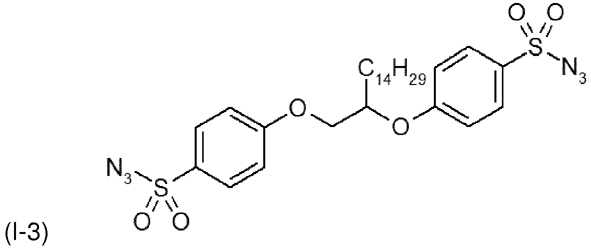
- 4,8-bis-(4-azidosulfonylphenoxymethyl)-tricyclo[5.2.1.0^{2.6}]decane (isomeric mixture), also designated POTCOP-SA, having the following formula: 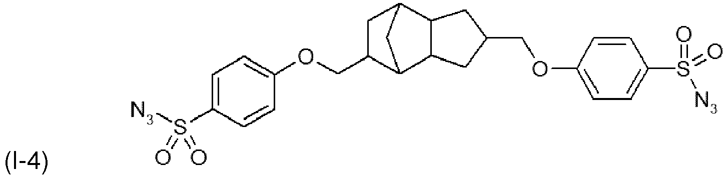
- 1,4-bis-(4-azidosulfonylphenoxymethyl)-cyclohexane, also designated POMCMOP-SA, having the following formula: 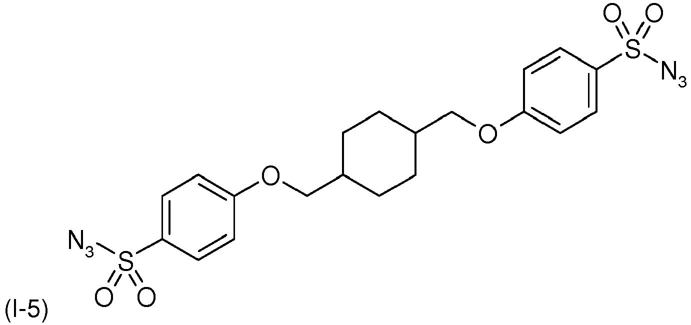
- 1,n-bis-(4-azidosulfonylphenyl)-polyethylene glycol ether, also designated POPEGOP-SA, having the following formula:

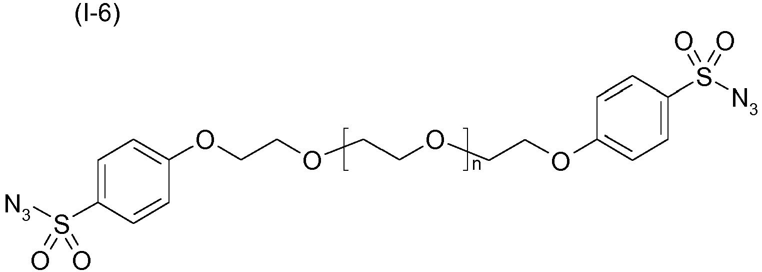

Preferably, in the compounds of the invention of type 2, L' is a functional group comprising an oxygen heteroatom. L' is -O-.

Preferred examples of a compound of type 2 of the present invention are:
- 4,4'-bis-(azidosulfonyl)-3-pentadecyl diphenylether, also designated PBSA, of the following formula: 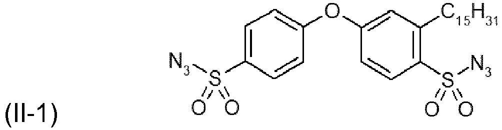
- a mixture of 2-fatty alkyl substituted DPO-BSA (*i.e.* a mixture of 4,4'-bis-(azidosulfonyl)-2-hexadecyl diphenylether and 4,4'-bis-(azidosulfonyl)-2-octadecyl diphenylether), also designated as H/ODPO-BSA, of the following formula:

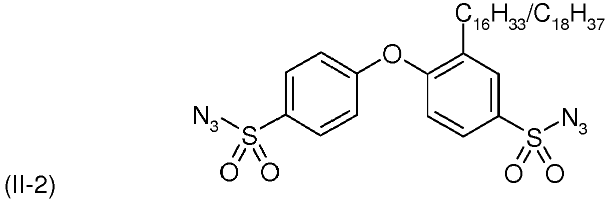

A preferred example of a compound of a mixed type of the present invention (both type 1 and type 2) is:
- 1,6-bis-(4-azidosulfonyl-3-pentadecylphenoxy)hexane, also designated PDPOHOPDP-SA, of the following formula:

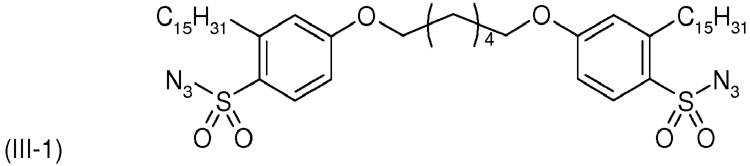

The compounds of the present invention can be prepared by reacting starting compounds A and B, optionally in the presence of an additional compound C. Compounds A and B are substituted aromatic compounds. They can be identical or different. They can bear sulfonylazide substituents. Alternatively, the sulfonylazide groups can be provided in one or more posterior reaction steps.

In particular, in order to produce compounds of type 1 having the above formula (I"), the starting compounds A and B are one and the same compound, and use can be made of a compound of formula Z-Sp"-Z as an additional compound C, wherein Sp" is as defined above, and Z is a reactive functional group.

More specifically, some of the compounds of type 1 can be prepared according to so-called route 1, comprising the following first step:

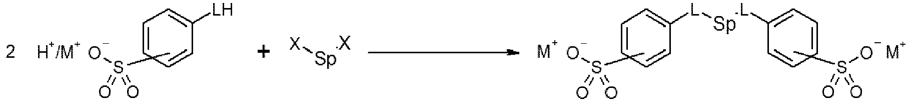

In the above scheme, M⁺ represents a metallic cation, such as for instance sodium or potassium. Sp is a spacer (denoted Sp" above) and L has a different meaning from formula (I).

According to one embodiment, L is selected from O, NH, or NR, where R is an alkyl or an aryl group, as defined above, and X is selected from Cl, Br, l, -NCO, -OC(=O)Cl, and -NR'C(=O)Cl, where R' is an alkyl or an aryl group, as defined above.

According to another embodiment, LH is SH or S⁻M⁺ and X is Cl, Br, or I.

This reaction can be performed in the presence of an inorganic base such as MOH, M₂CO₃, or in the presence of an organic base.

In case L is S, it is possible to then oxidize this linking group into SO₂ *e.g.* by treatment of the sulfide with hydrogen peroxide.

The first step is then followed by a second step of reacting the obtained compound with a chlorination agent. The chlorination agent can be for instance selected from thionylchloride, sulfurylchloride, phosphorylchloride, phosgene, oxalyl chloride, preferably thionylchloride.

The second step is then followed by a third step of reacting the obtained sulfonyl chloride compound with an azide source, preferably sodium azide. The second and third steps can be illustrated as follows:

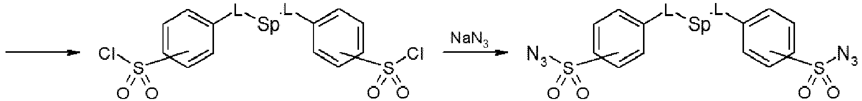

Route 1 makes it possible to obtain compounds of formula (I"), wherein Y is -O-, -NH-, -NR-, -OC(=O)NH-, -OC(=O)NR'-, -OC(=O)O-, -NHC(=O)NH-, -NHC(=O)O-, -NHC(=O)NR'-, -NRC(=O)NH-, -NRC(=O)O-, -NRC(=O)NR'-, -S-, and -SO₂-.

The same compounds of type 1 can be prepared according to so-called route 2, comprising the following step:

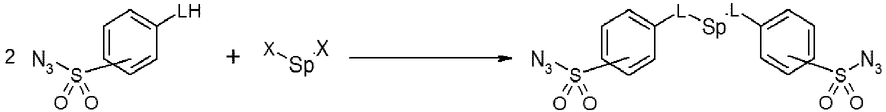

In the above scheme, LH, L, and X have the same meanings as in route 1. This reaction can be performed in the presence of an inorganic base such as MOH, M₂CO₃, or in the presence of an organic base.

Route 2 circumvents the need for any posterior chlorination or azidation step.

Other compounds of type 1 can be prepared according to so-called route 3, comprising the following first step:

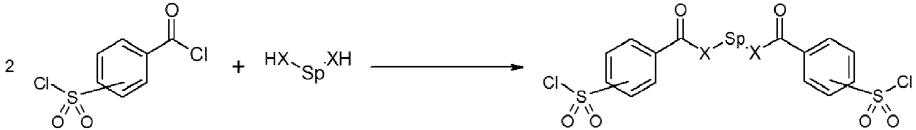

In the above scheme, X is O, NH, or NR, R being an alkyl or aryl group as defined above.

This reaction can be performed in the presence of an inorganic base such as MOH, M₂CO₃, or in the presence of an organic base.

The first step is then followed by a second step of reacting the obtained compound with sodium azide. The second step can be illustrated as follows:

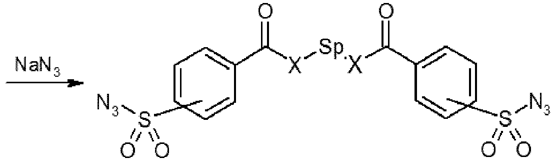

Route 3 makes it possible to obtain compounds of formula (I"), wherein Y is -C(=O)O-, -C(=O)NH- or -C(=O)NR-.

Other compounds of type 1 can be prepared according to so-called route 4, comprising the following first step:

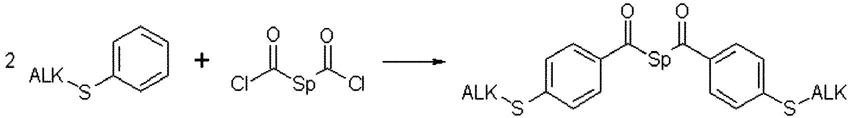

In the above scheme, ALK is an alkyl chain, such as a methyl group for instance.

This reaction can be performed in the presence of AlCl₃ or any other Lewis acid.

The first step is then followed by a second step of reacting the obtained compound with a mixture of Cl₂ and H₂O, and then by a third step of reacting the obtained sulfonyl chloride with an azide source like sodium azide. The second and third steps can be illustrated as follows:

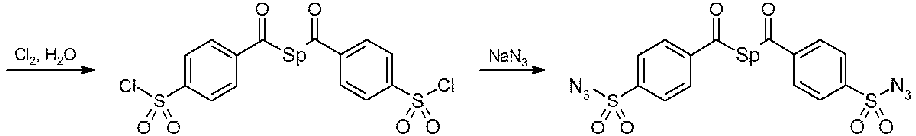

Route 4 makes it possible to obtain compounds of formula (I"), wherein Y is -C(=O)-.

Some of the compounds of type 2 can be prepared according to so-called route 5, comprising the following first step:

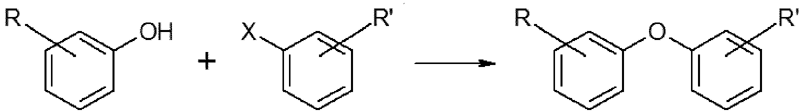

In this scheme, X is Cl, or Br, or I. R and R' are alkyl or aryl groups, as defined above. One of R and R' can be omitted; at least one of R and R' is or contains the above-defined spacer Sp or Sp'. Preferably, -R is -Sp-H or -R' is -Sp'-H.

This reaction step is preferably catalyzed by a transition metal catalyst, *e.g*. based on copper or palladium.

The first step is then followed by a second step of reacting the obtained compound with a chlorosulfonation agent. For instance, use may be made of at least four equivalents chlorosulfonic acid for direct chlorosulfonation, or at least two equivalents chlorosulfonic acid or sulfuric acid followed by a chlorination as described in route 1, or sulfurylchloride, etc.

The second step is then followed by a third step of reacting the obtained compound with an azide source like sodium azide. The second and third steps can be illustrated as follows:

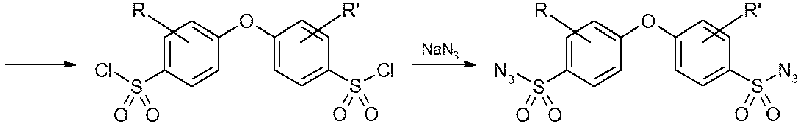

Route 5 thus makes it possible to prepare compounds of type 2 wherein L' is -O-.

Some of the compounds of type 2 can be prepared according to so-called route 6, comprising the following first and second steps:

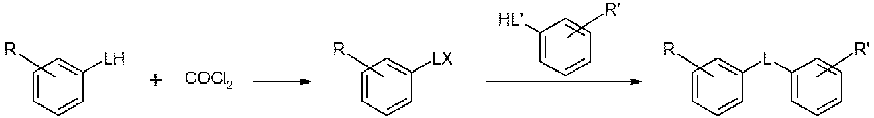

In this scheme, R and R' are alkyl or aryl groups, as defined above. One of R and R' can be omitted; at least one of R and R' is or contains the above-defined spacer Sp or Sp'. L has a different meaning from above in this scheme.

In the first step, LH is OH, NH₂ or -NR"H, where R" is an alkyl or aryl group having the same general definition as R and R'.

In the second step, LX is -OC(=O)Cl, -N=C=O or -NR"C(=O)Cl, R" being as defined above; and L'H is OH, NH2 or -NR"'H, where R'" is an alkyl or aryl group having the same general definition as R and R'.

In the compound obtained at the second step, L has a different meaning and can be -OC(=O)O-, -OC(=O)NH-, -OC(=O)NR"'-, -NHC(=O)O-, -NR"C(=O)O-, -NHC(=O)NH-, -NHC(=O)NR"'-, -NR"C(=O)NH- or -NR"C(=O)NR"'-, R" and R'" being as defined above.

The second step is followed by a third step of chlorosulfonation and a fourth step of reacting with sodium azide, which are analogous to the second and third step of route 5.

Some of the compounds of type 2 can be prepared according to so-called route 7, comprising the following first step:

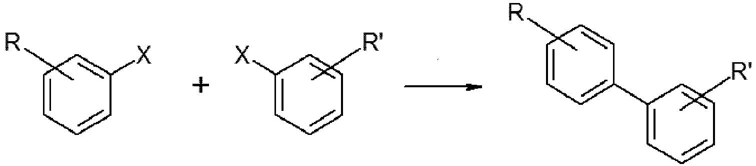

In this scheme, X is Cl, or Br or I; and R and R' are alkyl or aryl groups, as defined above. One of R and R' can be omitted; at least one of R and R' is or contains the above-defined spacer Sp or Sp'. This reaction step is preferably catalyzed with a transition metal catalyst, based e.g. on copper or palladium.

This first step is followed by a second step of chlorosulfonation and a third step of reacting with an azide source like sodium azide, which are analogous to the second and third step of route 5.

Route 7 makes it possible to obtain compounds of type 2, wherein L' in formula (II) is a covalent bond.

Some of the compounds of type 2 can be prepared according to so-called route 8, comprising the following first step:

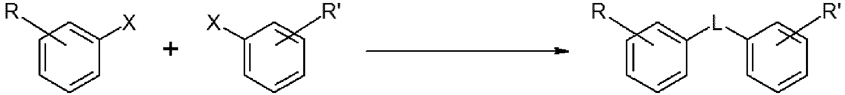

In this scheme, X is Cl, or Br or I; and R and R' are alkyl or aryl groups, as defined above; and L is -S- or -SO₂-.The reaction is performed in the presence of a compound M₂S, where M is a metallic element (for instance sodium or potassium) and in the presence of a catalyst, preferably a transition metal catalyst, e.g. based on copper.

An oxidation step is then performed if the -SO₂- linking group is desired *e.g.* by treatment of the sulfide with hydrogen peroxide.

Then, a second step of chlorosulfonation and a third step of reaction with an azide source like sodium azide are performed, in an analogous manner as in route 5.

Route 8 makes it possible to obtain compounds of type 2, wherein L' in formula (II) is -S- or -SO₂-.

Some of the compounds of type 1 can be prepared according to so-called route 9, comprising the following first step: the activation of a diol for a subsequent nucleophilic substitution, e.g. as a sulfonate like a mesylate or a tosylate.

The first step is then followed by a second step of a substitution of the activated diol with two equivalents of a hydroxyarene sulfonic acid, e.g. 4-hydroxybenzene sulfonic acid.

The second step is then followed by a third step of an activation of the sulfonic acid groups for the azidation, e.g. as a sulfonyl chloride by treating the sulfonic acid with thionyl chloride.

The third step is then followed by a fourth step of the sulfonyl azide formation by reacting the activated sulfonic acid with an azide, e.g. sodium azide.

Route 9 makes it possible to obtain compound of formula (I") wherein Y is a diol like 4,8-bis hydroxymethyl tricyclo[5.2.1 .0^{2.6}]decane to form POTCOP-SA or 1,4-bis hydroxymethyl cyclohexane to form POMCMOP-SA.

In order to prepare compounds of the invention where the aromatic building blocks Ar and Ar' are not based on phenyl groups but rather on different aryl groups, similar reaction routes may be employed by analogy.

One or more compounds of the invention can be formulated in a cross-linker composition. According to one embodiment, this cross-linker composition is devoid of any phlegmatizing agent. According to an alternative embodiment, this cross-linker composition comprises a phlegmatizing agent. A phlegmatizing agent is defined as an inert compound which reduces the explosiveness of the bissulfonylazide compound, according to the tests defined in the UN manual for tests and criteria.

Phlegmatizing agents can be organic or inorganic compounds. Organic compounds can be solvents or solids like e.g. Irganox® 1010. Inorganic compounds can be e.g. diatomaceous earth or silica gel.

The amount of phlegmatizing agent used in the present invention can be varied over a wide range, depending on how much phlegmatizing agent is required to ensure safe handling and use of the compounds of the invention and to allow a transport classification as non-explosive material. In particular, the amount of phlegmatizing agent in the compositions of the invention can be from 0 to 50% (w/w), preferably from 0 to 40% (w/w), more preferably from 0 to 30% (w/w), even more preferably from 0 to 20% (w/w), and most preferably from 0 to 10% (w/w).

The compounds and compositions of the invention are useful in the manufacture of polymers such as polyolefins (in particular polypropylene and polyethylene), in the cross-linking of polymers, and in the manufacture of synthetic rubber.

### EXAMPLES

The following examples illustrate the present invention without limitation.

### Example 1: synthesis of POHOP-SA

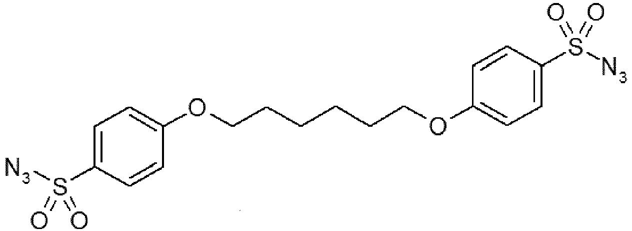

### Step 1: 1,6-bis-(4-sulfophenoxy)-hexane disodium salt (POHOP-SONa)

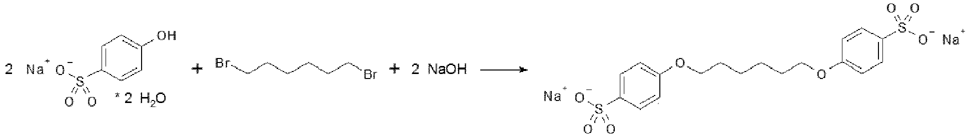

A solution of 320 sodium hydroxide in 480 g water was added slowly at 25°C to a solution of 1858 g sodium 4-hydroxybenzen e sulfonate and 12.8 g tetrabutylammonium bromide in 3120 g water. To this mixture 976 g 1,6-dibromohexane was added within 1 h. The mixture was heated to 100°C and stirred for 15 h. The thick slurry was diluted with 400 ml water and stirred for another 7 h at 100°C. The suspension was diluted wi th 11.6 L water and stirred at 100°C for 8 h and then cooled to room temperatur e for filtration. The solids were washed with 4 L water followed by 4 L acetone and dried at 50°C. 1599 g of a white solid with an assay of 92.5% (w/w, HPLC) was isolated, with chemical yield of 80%.

### Analytical data:

Melting point >345°C; 1H-NMR (300 MHz in D₂O): 7.65 ppm (d, 4H), 6.97 ppm (d, 4H), 4.02 ppm (t, 4 H), 1.70 ppm (broad quintet, 4 H), 1.40 ppm (broad, 4 H).

### Step 2: 1,6-bis-(4-chlorosulfonylphenoxy)-hexane (POHOP-SC)

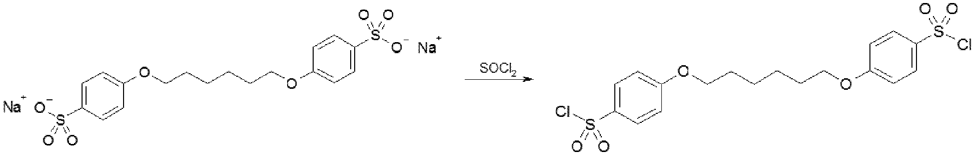

A mixture of 1471 g POHOP-SONa, 7 kg thionyl chloride, and 7 g dimethylformamide (DMF) was heated to 75°C for 17 h . 3.5 kg thionyl chloride were removed by distillation at 50°C and 100 mbar and the distillation was continued after the addition of three times each 2 L toluene until a distillation residue of 2.3 kg was obtained. It was diluted with 1.5 L toluene and cooled to 17°C. The solids were filtered off and washed with 1.5 L toluene. The wet filter cake was slurried in 2.5 L water in order to removed salts and residual traces of thionyl chloride. The solids were filtered, washed with 2.5 L water, and dried at 40°C. 1329 g of a white solid was obtained. The chemical yield based on the mass of the product is 99%.

### Analytical data:

Melting point: 126 - 128°C; 1H-NMR (300 MHz in CDCl 3): 7.97 ppm (d, 4H), 7.05 ppm (d, 4H), 4.11 ppm (t, 4 H), 1.90 ppm (broad quintet, 4 H), 1.60 ppm (broad, 4 H).

### Step 3: 1,6-bis-(4-azidosulfonylphenoxy)-hexane (POHOP-SA)

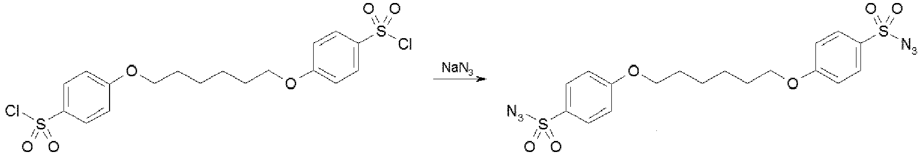

A solution of 406 g sodium azide in 3.25 L water was added within 15 min to a solution of 1328 g POHOP-SC in 16.25 L acetone. The mixture was stirred at room temperature for 53 h and then diluted with 14.8 L water in order to dissolve salts. The solids were recovered by filtration and washed with 9 L water. After 2 days of drying at 40°C, 1301 g of a white solid was isolated. The chemical yield was 98% based on an assay determination of 96.6% w/w by NMR.

### Analytical data:

Melting point: 93 - 95°C; 1H-NMR (300 MHz in CD2CI2): 7.91 ppm (d, 4H), 7.10 ppm (d, 4H), 4.12 ppm (t, 4 H), 1.90 ppm (broad quintet, 4 H), 1.60 ppm (broad, 4 H).

### Example 2: safety tests for POHOP-SA in comparison with DPO-BSA:

Neat POHOP-SA is neither shock sensitive in the BAM-fallhammer test with an impact energy of 40 J, nor friction sensitive in the BAM-friction apparatus with a friction force of 360 N.

Neat POHOP-SA is explosive according to the Koenen test with a bore hole diameter of 2 mm according to the UN Manual of Tests and Criteria, Part l, Revision V, United Nations, 2009, p.33 ff (section 11.5.1).

POHOP-SA with 5 % Irganox® 1010 is still explosive in the Koenen test at 2 mm bore hole diameter but not anymore in a mixture containing 10 % Irganox® 1010.

For comparison, according to safety tests, POHOP-SA requires only 10% Irganox® 1010 for sufficient phlegmatization whereas the comparative example DPO-BSA requires more than 33% Irganox® 1010.

### Example 3: synthesis of PODOP-SA

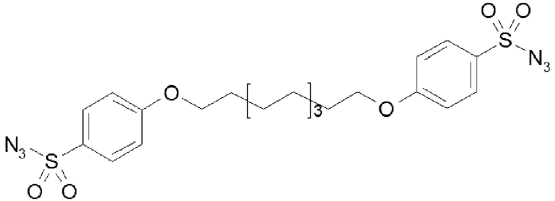

### Step 1: 1,10-bis-(4-sulfophenoxy)-decane disodium salt (PODOP-SONa)

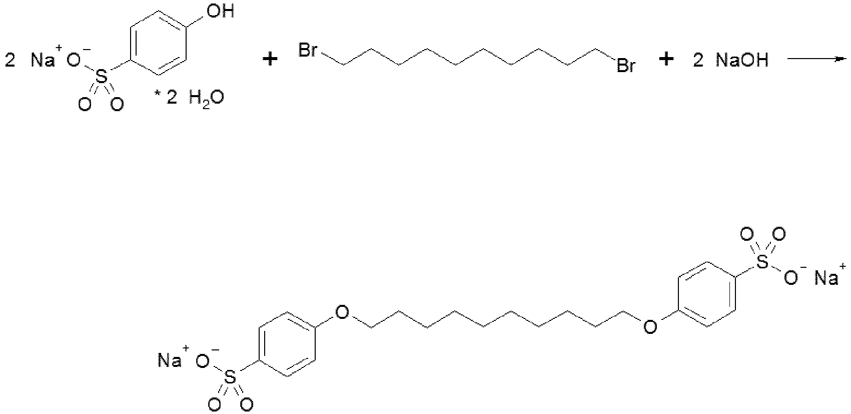

A solution of 40 g sodium hydroxide in 60 g water was added slowly at 25°C to a thin suspension of 232.2 g sodium 4-hydro xybenzene sulfonate in a mixture of 198 g water and 218 g DMSO. To this mixture, 150 g of 1,10-dibromodecane was added within 20 min. The mixture was refluxed at 113°C for 24 h, and then cooled to room temperature for filtration. The solids were washed with 0.9 L water followed by 1 L acetone and dried at 50°C. 244.6 g of a white solid with a purity of 94.1 area-% (HPLC) was isolated with a chemical yield of 87%.

### Analytical data:

Melting point >350°C; 1H-NMR (300 MHz in DMSO): 7.4 9 ppm (d, 4H), 6.84 ppm (d, 4H), 3,95 ppm (t, 4 H), 1.70 ppm (m, 4 H), 1.25 -1.45 ppm (broad, 12 H).

### Step 2: 1,10-bis-(4-chlorosulfonylphenoxy)-decane (PODOP-SC)

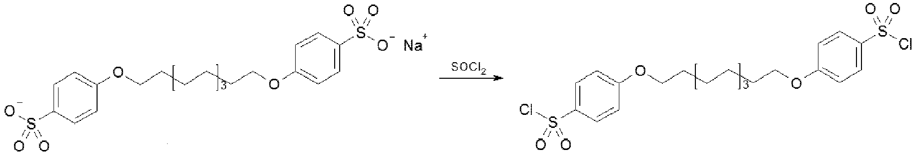

A mixture of 244.1 g PODOP-SONa, 821 g thionyl chloride, and 2.4 g DMF was heated to 75°C for 7 h. 330 g thionyl chloride were removed by distillation at 50°C and 100 mbar. The residue was diluted with 900 mL petrolether 30/50 at 25°C. The solids were filtered off after 24 h and washed with 500 ml petrolether 30/50. The wet filter cake was slurried in 910 mL water in order to remove salts and residual traces of thionyl chloride. The solids were filtered, washed with 0.5 l water, and dried at 40°C. 228.3 g of a light brown solid was obtained. The chemical yield based on the mass of the product was 98%.

### Analytical data:

Melting point: 63 - 65°C; 1H-NMR (300 MHz in CDCl3) : 7.98 ppm (d, 4H), 7.05 ppm (d, 4H), 4.08 ppm (t, 4 H), 1.85 ppm (broad quintet, 4 H), 1.30 - 1.60 ppm (broad, 12 H).

### Step 3: 1,10-bis-(4-azidosulfonylphenoxy)-decane (PODOP-SA)

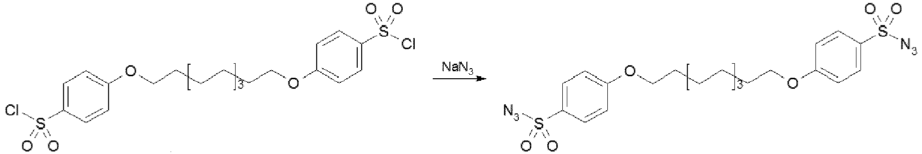

A solution of 69.7 g sodium azide in 609 mL water was added within 30 min to a solution of 227.7 g PODOP-SC in 3.04 L acetone. The mixture was stirred at room temperature for 24 h and then diluted with 2.53 L water in order to dissolve salts. The solids were recovered by filtration and washed with 0.6 L water. After 2 days of drying at 40°C, 210 g of a I ight brown solid was isolated. 208 g of the crude product was dissolved in 330 g acetone. 1 g activated charcoal and 2 g diatomaceous earth were added and the mixture was filtered at 55°C. The filtrate was cooled to 26°C when cryst allization started with a temperature rise to 29°C. At this temperature, 208 mL water was added to the mixture and the solids were recovered by filtration and washed with 208 mL water. Drying at 40°C resulted in 204.7 g off-white PDOOP-SA with 98.4 area-% purity. The chemical yield was 88%.

### Analytical data:

Melting point: 89 - 91°C; 1 H-NMR (300 MHz in DMSO): 7.93 ppm (d, 4H), 7.23 ppm (d, 4H), 4.11 ppm (t, 4 H), 1.74 ppm (broad quintet, 4 H), 1.25 - 1.45 ppm (broad, 12 H).

### Example 4: safety tests for PODOP-SA in comparison with DPO-BSA

Neat PODOP-SA is neither shock sensitive in the BAM-fallhammer test with an impact energy of 40 J nor friction sensitive in the BAM-friction apparatus with a friction force of 360 N.

Neat PODOP-SA is not explosive according to the Koenen test with a bore hole diameter of 2 mm.

According to the time/pressure test, it does not deflagrate quickly.

According to these safety tests, PODOP-SA is not explosive, does not have to be included in class 1 according to transport regulations and thus does not have to be phlegmatized at all.

### Example 5: synthesis of POMCMOP-SA

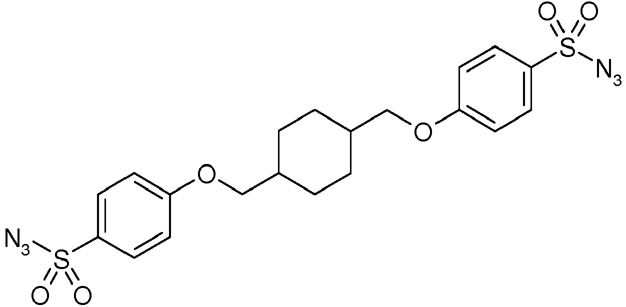

### Step 1: 1,4-bis-(methane sulfonyloxymethyl)-cyclohexane (MCM-OMs)

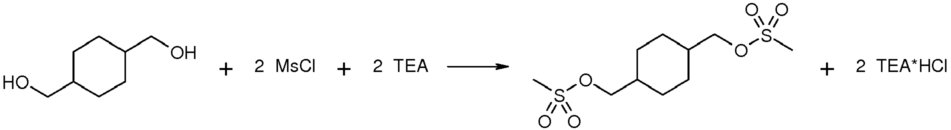

To a cold mixture of 46.9 g 1,4-bis-(hyroxymethyl) cyclohexane (mixture of cis and trans isomers), 79.3 g triethylamine, and 350 ml THF was added 84.1 g methane sulfonyl chloride slowly at max. 25°C. 200 ml THF and 10 ml methane sulfonyl chloride were added and the mixture was quenched with 400 ml water. The solids were recovered by filtration and dried at 40°C to yield 78.2 g of a white solid (80% chemical yield; mixture of two isomers). A second crop of 8.5 g (9% chemical yield) was isolated from the mother liquor as crystals that formed upon standing over night. This minor product was determined by X-ray crystallography to be the cis-isomer. The major compound of the main fraction was the trans-isomer (82:18).

### Analytical data:

¹H-NMR (300 MHz in CDCN): 4.14/4.04 ppm (d, 4H), 3,02 ppm (s, 6H), 1.1 to 2.2 ppm (several signals, 10 H).

### Step 2: 1,4-bis-(4-sulfophenoxymethyl)-cyclohexane disodium salt POMCMOP-SONa

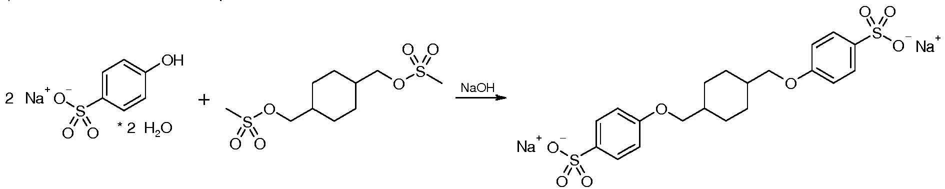

A suspension of 69.3g sodium 4-hydroxybenzene sulfonate in a solution of 10.8 g sodium hydroxide in 230 ml water was added at 25°C to a solution of 38.7 g MCM-OMs in 220 ml DMSO. The mixture was heated to 120°C and stirred for a total of 28 h. The slurry was cooled to 5°C and filtered. The solids were washed with acetone and re-crystallized from 2 l water. A first crop of 27.4 g (43% chemical yield) was isolated by filtration at 35°C and a second crop of 6.4 g (10% chemical yield) at 5°C. The isol ated product contained only one isomer, due to the yield in comparison to the 82:18 composition of the starting material isomer mixture most likely the trans-isomer.

### Analytical data:

¹H-NMR (300 MHz in D₂O): 7.66 ppm (d, 4H), 7.00 ppm (d, 4H), 3.89 ppm (d, 4 H), 1.85 ppm (d, 4 H), 1.73 (broad, 2H), 1.60 ppm (quintet, 4 H).

### Step 3: 1,4-bis-(4-chlorosulfonylphenoxymethyl)-cyclohexane (POMCMOP-SC)

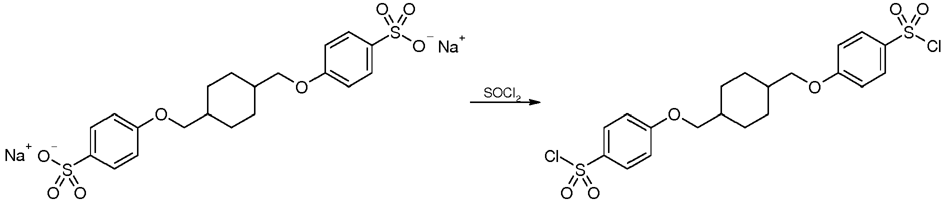

A mixture of 27.4 g POMCMOP-SONa, 588 g thionyl chloride, and 0.8 g dimethylformamide (DMF) was heated to 50°C for 6 h and stirred at room temperature for 60 h. The excess of thionyl chloride was removed by distillation at 50°C in vacuum. The remaining solid was stirred in a mixture of dichloromethane and ice water. A filtration yielded 17.3 g (64% chemical yield) white solid. The organic phase was boiled down to a suspension which was heated until the solids dissolved and cooled for the crystallization of a second crop of 3.1 g (11% chemical yield) product:

### Analytical data:

¹H-NMR (300 MHz in CD₂Cl₂): 8.00 ppm (d, 4H), 7.09 ppm (d, 4H), 3.95 ppm (d, 4 H), 2.03 ppm (d, 4 H), 1.91 (broad, 2H), 1.22 ppm (quintet, 4 H).

### Step 4: 1,4-bis-(4-azidosulfonylphenoxymethyl)-cyclohexane (POMCMOP-SA)

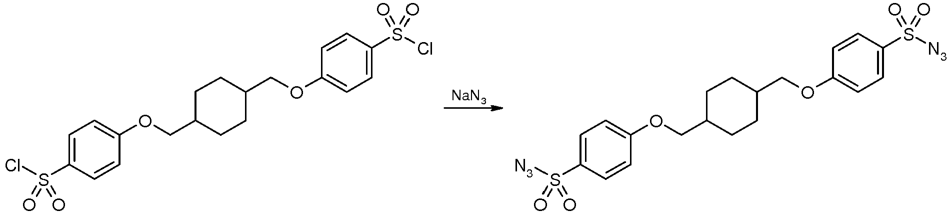

A solution of 1 g sodium azide in 11 ml water was added to a solution of 3.1 g POMCMOP-SC in 20 ml acetone. The mixture was diluted with 20 ml acetone and stirred at room temperature for 24 h. The mixture was stirred for additional 12 h after the addition of 0.1 g sodium azide. The solids were recovered by filtration, washed with water, and dried at 20 mbar. The product was isolated in quantitative yield.

### Analytical data:

Melting point: 144°C (DSC); ¹H-NMR (300 MHz in CD2CI2): 7.91 ppm (d, 4H), 7.10 ppm (d, 4H), 3.94 ppm (d, 4 H), 2.03 ppm (d, 4 H), 1.90 (broad, 2H), 1.23 ppm (quintet, 4 H).

### Example 6: Limited safety tests for POMCMOP-SA in comparison with DPO-BSA

Neat POMCMOP-SA releases 1306 J/g energy with an onset at 168°C in a DSC-measurement. It is neither shock sensitive in the BAM-fallhammer test with an impact energy of 40 J, nor friction sensitive in the BAM-friction apparatus with a friction force of 360 N.

### Comparative example: explosive properties of DPO-BSA

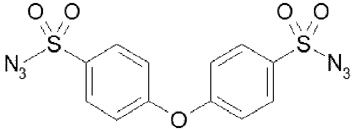

Neat DPO-BSA is shock sensitive in the BAM-fallhammer test with an impact energy of 15 J (like the commercial explosive TNT) but not friction sensitive in the BAM-friction apparatus with a friction force of 360 N.
Neat DPO-BSA is explosive according to the Koenen test with a bore hole diameter of 2 mm.

According to safety tests, DPO-BSA requires more than 33% Irganox® 1010 for sufficient phlegmatization.

### Example 7: Application of POHOP-SA and PODOP-SA for cross-linking of polypropylene

Polypropylene (Moplen® HP 500 N) was partially cross-linked with POHOP-SA or PODOP-SA. For this a dry blend of POHOP-SA with Irganox® 1010 in the mass ratio 90:10 or PODOP-SA without phlegmatizing agent powder was prepared with polypropylene. In the comparative experiments DPO-BSA in a mixture with Irganox® 1010 in the mass ratio 24:76 was used in equimolar amounts of DPO-BSA compared to POHOP-SA and PODOP-SA with polypropylene (Moplen® HF 500). A mixture of polypropylene and the dry blend was passed through a twin screw extruder at 100 to 230°C. The extruded polymer was cooled in a water bath and chopped into pieces.

The mechanical characteristic data (tensile modulus, tensile stress, tensile strain) of the modified polypropylene were measured with a testing machine UPM 1455 from the company Zwick. It is apparent from the results given in the tables 1 and 2 that the new cross linking agents POHOP-SA and PODOP-SA show similar performance as DPO-BSA in the comparative example. The increase of tensile modulus is measured between a non-cross-linked and a cross-linked polypropylene. The tensile modulus, tensile stress and tensile strain are measured at a frequency of 0.04 rad/second.

**Table 1: Test results for PP treated with 0.329 mmol cross linker per 1 kg PP**

| Cross linker | POHOP-SA 90% | PODOP-SA 100% | DPO-BSA 24% (reference cross-linker) |
|---|---|---|---|
| Tensile modulus [MPa] | 1598 | 1605 | 1661 |
| Increase of tensile modulus [%] | 9 | 10 | 9 |
| Tensile stress [MPa] | 35 | 36 | 36 |
| Tensile strain [%] | 9 | 9 | 8 |

**Table 2: Test results for PP treated with 0.657 mmol cross linker per 1 kg PP**

| Cross linker | POHOP-SA 90% | PODOP-SA 100% | DPO-BSA 24% (reference cross-linker) |
|---|---|---|---|
| Tensile modulus [MPa] | 1675 | 1683 | 1737 |
| Increase of tensile modulus [%] | 14 | 15 | 14 |
| Tensile stress [MPa] | 36 | 36 | 37 |
| Tensile strain [%] | 9 | 9 | 8 |

The Charpy notch impact strength was tested, according to DIN-EN ISO 179, for polypropylene and polypropylene modified with POHOP-SA or PODOP-SA (See table 3). Both cross-linkers result in an increased notch impact strength versus non-cross-linked polypropylene, a higher cross-linker ratio to polypropylene resulting in an increased notch impact strength.crosslink. The effect is more pronounced for PODOP-SA than for POHOP-SA.

**Table 3: Charpy notch impact strength for PP treated with POHOP-SA or PODOP-SA [kJ/m²]**

| | | | | |
|---|---|---|---|---|
| amount of phlegmatizing agent [mmol/kg] | 0 | 0.329 | 0.657 | 2.629 |
| POHOP-SA 90% with lrganox® 1010 | 2,7 | 2,8 | 2,9 | 3,9 |
| PODOP-SA | 2,7 | 3,0 | 3,3 | 4,1 |

### Example 8: Application of POHOP-SA and PODOP-SA for cross-linking of high density polyethylene

High density polyethylene (Lupolen® 5031 L Q 449 K - HDPE) was partially cross-linked with POHOP-SA or PODOP-SA. For this a dry blend of POHOP-SA with Irganox® 1010 in the mass ratio 90:10 or PODOP-SA without phlegmatizing agent was prepared with HDPE powder. In the comparative experiments DPO-BSA in a mixture with Irganox® 1010 in the mass ratio 24:76 was used in equimolar amounts of DPO-BSA compared to POHOP-SA and PODOP-SA with HDPE. A mixture of HDPE and the dry blend was passed through a twin screw extruder at 100 to 200°C. The extruded polymer was cooled in a water bath and chopped into pieces.

As for polypropylene, it is apparent, from the results given in the table 4, that the new cross linking agents POHOP-SA and PODOP-SA show the similar performance as DPO-BSA in the comparative example.

**Table 4: Test results for HDPE treated with 2.629 mmol cross linker per 1 kg HDPE**

| Cross linker | POHOP-SA 90% | PODOP-SA 100% | DPO-BSA 24% (reference cross-linker) |
|---|---|---|---|
| Tensile modulus [MPa] | 907 | 861 | 886 |
| Increase of tensile modulus [%] | 2 | 7 | 5 |
| Tensile stress [MPa] | 25 | 24 | 24 |
| Tensile strain [%] | 10 | 10 | 10 |

The rheological properties of partially cross-linked HDPE were measured at 150°C in a nitrogen atmosphere with a plate rheometer of the company Rheometrics (plate diameter 25 mm; plate distance 1 mm).

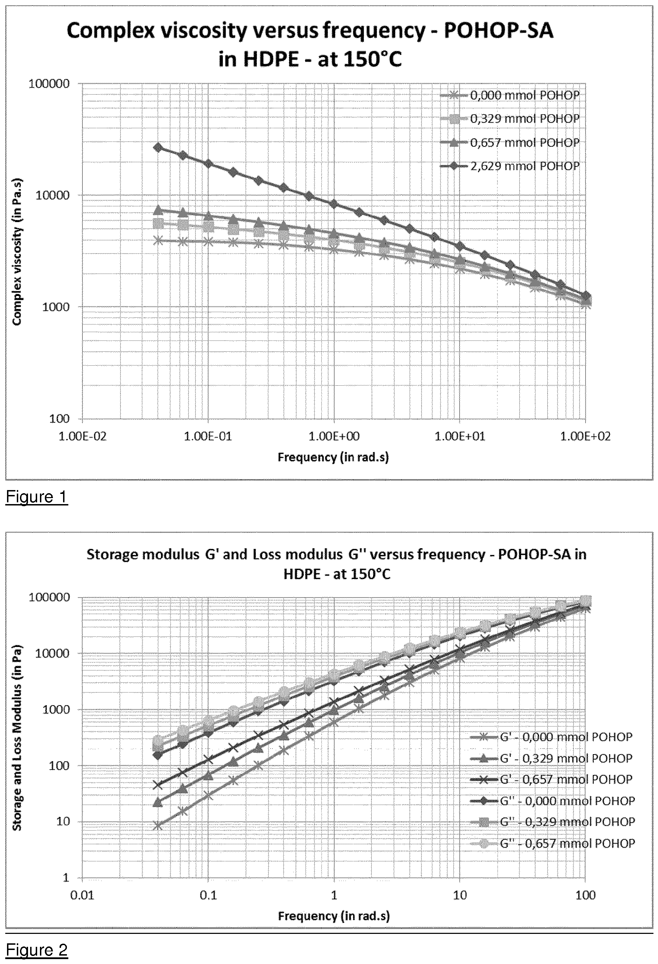

Compared with non-cross-linked HDPE, the complex viscosity increases with increasing molar amount of POHOP-SA per 1 kg of HDPE (see figure 1).Compared with non-cross-linked HDPE, the complex viscosity was increased by a factor of almost 7 at a frequency of 0.04 rad/s, with 2.629 mmol POHOP-SA per 1 kg of HDPE

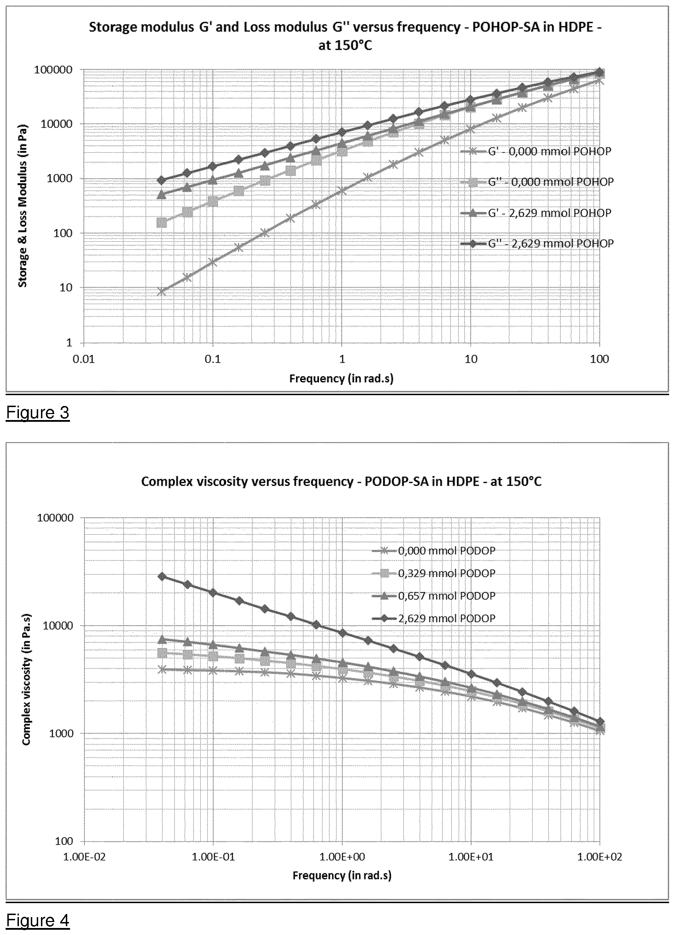

Making reference to Figures 2 and 3, at low levels of 0.329 and 0.657 mmol POHOP-SA for 1 kg HDPE the loss modulus (G") increased moderately whereas the storage modulus (G') increased significantly (figure 2). The increase is very significant with 2.629 mmol POHOP-SA for 1 kg HDPE (figure 3).

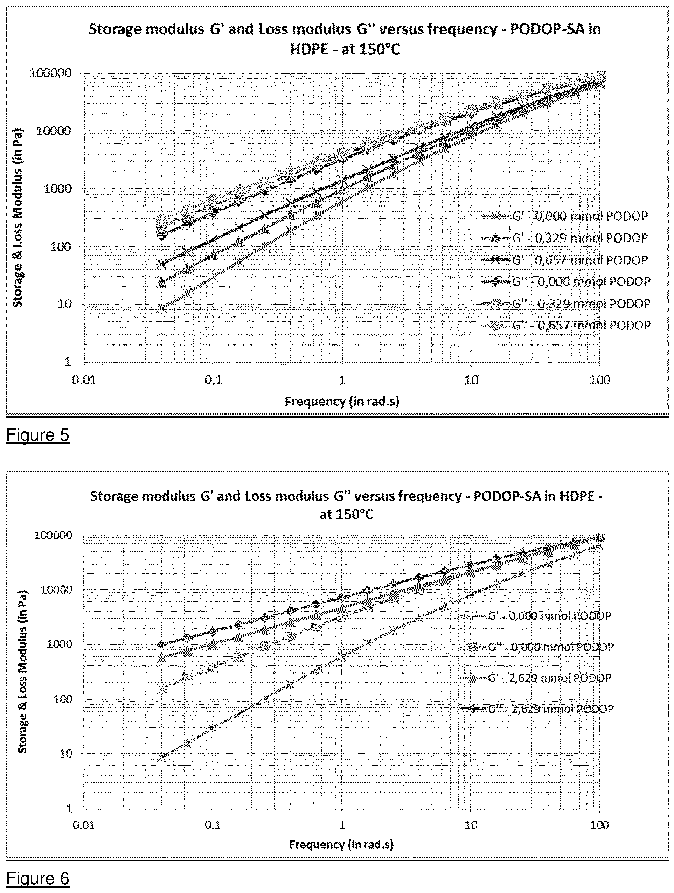

The results with PODOP-SA were very similar to POHOP-SA (see figures 4, 5, and 6).

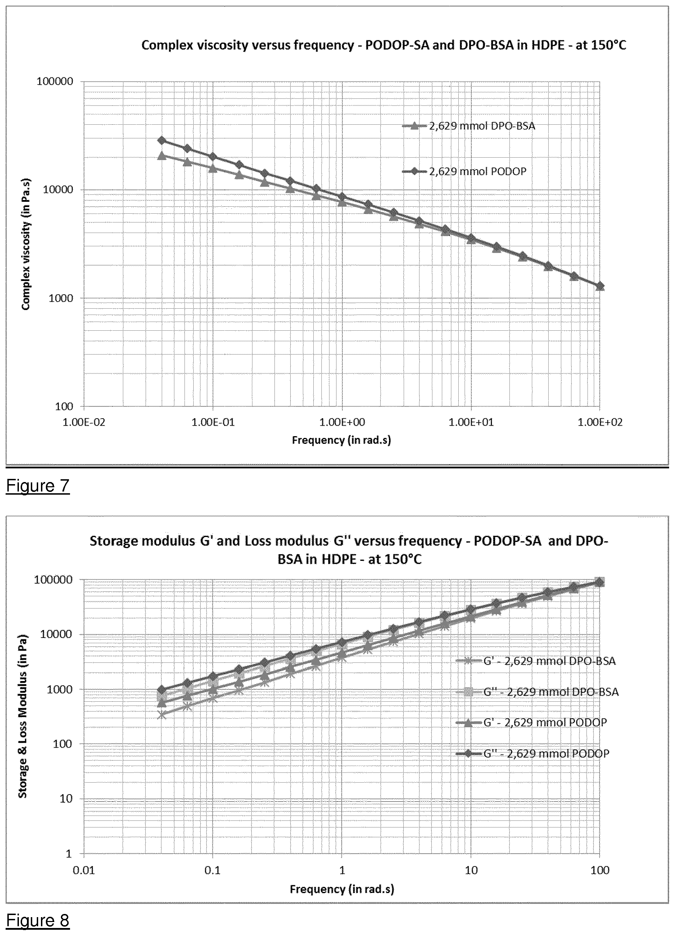

The effect of PODOP-SA on the complex viscosity was greater than that of the reference cross-linker DPO-BSA at 2.629 mmol cross-linker for 1 kg HDPE (figure 7).

The effect of PODOP-SA on the loss and storage modulus was greater than that of the reference cross-linker DPO-BSA at 2.629 mmol cross-linker for 1 kg HDPE (figure 8).

In comparison with DPO-BSA, the same molar amount of new azides to HDPE provides a higher value of loss and/or storage modulus. For a given target value of loss and/or storage modulus, a smaller molar amount of new azides is sufficient compared to DPO-BSA.

The melt strength of HDPE and partially cross-linked HDPE was measured with a stretch tester of the type Rheotens® in combination with a high pressure capillary rheometer of the type Rheograph® 2000 both of the company Göttfert. The melt strand was extruded at a constant speed of 11.3 mm/s through a nozzle. The stretching was measured with the Rheotens® equipment which was set up at 125 mm distance from the nozzle. The pull-off roll was accelerated with 2.4 mm/S² until the melt strand ripped off.

HDPE without cross-linking has a pull-off force of 0.013 N and the pull-off speed as a relative measure for the melt strength is 320 to 360 m/s.

Both, 2.629 mmol/kg POHOP-SA and PODOP-SA increase the pull-off force by a factor 3 to 0.04 N.

POHOP-SA increases the pull-off speed by a factor 2. With PODOP-SA the pull-off speed remains at the same level as without cross-linking.

POHOP-SA has an additional advantage as the processing of HDPE can be improved to significantly higher speeds.

## Claims

1. A compound of the general formula
(I) (N₃O₂S-Ar-Y)₂Sp",
wherein:
- Ar is a phenyl group substituted or not; and
- Sp" is an aliphatic linear, branched or cyclic chain having at least two carbon atoms,
- Y is selected from -O-, -C(=O)O, -OC(=O)-

2. The compound according to claim 1, in which Y is -O-; and the organic chain Sp" is an aliphatic linear, branched or cyclic chain which preferably comprises at least 3, preferably at least 4, more preferably at least 5, and most preferably from 6 to 16 carbon atoms.

3. The compound of any one of claims 1 to 2, which is:
- the compound 1,6-bis-(4-azidosulfonylphenoxy)-hexane or the compound 1,10-bis-(4-azidosulfonylphenoxy)-decane or
- the compound 1,2-bis-(4-azidosulfonylphenoxy)-hexadecane or
- an isomeric mixture of 4,8-bis-(4-azidosulfonylphenoxymethyl)-tricyclo[5.2.1.0^{2.6}]decane or
- the compound 1,4-bis-(4-azidosulfonylphenoxymethyl)-cyclohexane or
- the compound 1,n-bis-(4-azidosulfonylphenyl)-polyethylene glycol ether, or
- 1,6-bis-(4-azidosulfonyl-3-pentadecylphenoxy)hexane.

4. A compound of the general formula 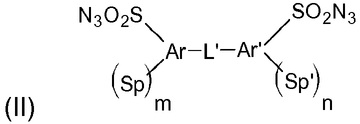 wherein:
- Ar and Ar' are phenyl groups ;
- L' is -O-;
- m is 1 and n is 0; or m is 0 and n is 1;
- Sp and Sp' are organic chains having at least two carbon atoms, wherein Sp and/or Sp' is an aliphatic linear, branched or cyclic chain comprising at least 3 carbon atoms.

5. The compound of claim 4, in which Sp and/or Sp' is an aliphatic linear, branched or cyclic chain comprising at least 4, more preferably at least 5 and most preferably from 6 to 16 carbon atoms.

6. The compound of any one of claims 4 to 5, which is :
- the compound 4,4'-bis-(azidosulfonyl)-3-pentadecyl diphenylether or
- 4,4'-bis-(azidosulfonyl)-2-hexadecyl diphenylether and/or 4,4'-bis-(azidosulfonyl)-2-octadecyl diphenylether.

7. A composition comprising the compound according to any one of claims 1 to 6, and containing at most 50% (w/w), preferably at most 40% (w/w), more preferably at most 30% (w/w), even more preferably at most 20% (w/w), most preferably at most 10% (w/w) of a phlegmatizing agent or which is without phlegmatizing agent.

8. The use of the compound of the general formula
(I) (N₃O₂S-Ar-Y)₂Sp",
wherein:
- Ar is a phenyl group substituted or not; and
- Sp" is an aliphatic linear, branched or cyclic chain having at least two carbon atoms,
- Y is selected from -O-, -C(=O)O, -OC(=O)-,
or of the general formula: 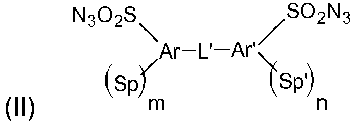 wherein:
- Ar and Ar' are aromatic building blocks;
- L' is -O-;
- m is 1 and n is 0; or m is 0 and n is 1;
Sp and Sp' are organic chains having at least two carbon atoms, wherein Sp and/or Sp' is an aliphatic linear, branched or cyclic chain comprising at least 3 carbon atoms,
as a cross-linker.

9. The use according to claim 8 of the compound of the general formula (I) or (II) as defined in any one of claims 1 to 6, or of the composition as defined in claim 7.

10. The use according to any one of claims 8 to 9, as a cross-linker in the manufacture of polymers.

11. The use according to any one of claims 8 to 10, as a cross-linker in the manufacture of polyolefins, polyolefin copolymers and blends of polyolefins, preferably in the manufacture of polypropylene or polyethylene.

12. The use according to any one of claims 8 to 11, as a cross-linker in the manufacture of elastomers, elastomer copolymers and blends of elastomers, preferably in the manufacture of EPM, EPDM and EPM-polyolefin blends.

## Patentansprüche

1. Verbindung der allgemeinen Formel
(l) (N₃O₂S-Ar-Y)₂Sp"',
wobei:
- Ar eine Phenylgruppe ist, die substituiert oder unsubstituiert ist; und
- Sp" eine aliphatische lineare, verzweigte oder cyclische Kette mit zumindest zwei Kohlenstoffatomen ist,
- Y aus -O-, -C(=O)O, -OC(=O)- ausgewählt ist.

2. Verbindung nach Anspruch 1, wobei Y -O- ist; und die organische Kette Sp" eine aliphatische lineare, verzweigte oder cyclische Kette ist, die vorzugsweise zumindest 3, vorzugsweise zumindest 4, mehr bevorzugt zumindest 5 und am meisten bevorzugt 6 bis 16 Kohlenstoffatome umfasst.

3. Verbindung nach einem der Ansprüche 1 bis 2, die ist:
- die Verbindung 1,6-Bis-(4-azidosulfonylphenoxy)hexan oder die Verbindung 1,10-Bis-(4-azidosulfonylphenoxy)decan oder
- die Verbindung 1,2-Bis-(4-azidosulfonylphenoxy)hexadecan oder
- eine isomerische Mischung von 4,8-Bis-(4-azidosulfonylphenoxymethyl)tricyclo[5.2.1.0^{2,6}]decan oder
- die Verbindung 1,4-Bis-(4-azidosulfonylphenoxymethyl)cyclohexan oder
- die Verbindung 1 ,n-Bis-(4-azidosulfonyl)polyethylenglykolether oder
- 1,6-Bis-(4-azidosulfonyl-3-pentadecylphenoxy)hexan.

4. Verbindung der allgemeinen Formel 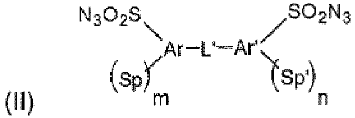 wobei:
- Ar und Ar' Phenylgruppen sind;
- L' -O- ist;
- m 1 ist und n 0 ist; oder m 0 ist und n 1 ist;
- Sp und Sp' organische Ketten mit zumindest zwei Kohlenstoffatomen sind, wobei Sp und/oder Sp' eine aliphatische lineare, verzweigte oder cyclische Kette ist, die zumindest drei Kohlenstoffatome umfasst.

5. Verbindung nach Anspruch 4, wobei Sp und/oder Sp' eine aliphatische lineare, verzweigte oder cyclische Kette ist, die zumindest 4, mehr bevorzugt zumindest 5 und am meisten bevorzugt 6 bis 16 Kohlenstoffatome umfasst.

6. Verbindung nach einem der Ansprüche 4 bis 5, die ist:
- die Verbindung 4,4'-Bis-(azidosulfonyl)-3-pentadecyldiphenylether oder
- 4,4'-Bis-(azidosulfonyl)-2-hexadecyldiphenylether und/oder 4,4'-Bis-(azidosulfonyl)-2-octadecyldiphenylether.

7. Zusammensetzung, die die Verbindung nach einem der Ansprüche 1 bis 6 umfasst und höchstens 50 % (Gew./Gew.), vorzugsweise höchstens 40 % (Gew./Gew.), mehr bevorzugt höchstens 30 % (Gew./Gew.), noch mehr bevorzugt höchstens 20 % (Gew./Gew.), am meisten bevorzugt höchstens 10 % (Gew./Gew.) eines Phlegmatisierungsmittels umfasst oder die kein Phlegmatisierungsmittel umfasst.

8. Verwendung der Verbindung der allgemeinen Formel
(l) (N₃O₂S-Ar-Y)₂Sp",
wobei:
- Ar eine Phenylgruppe ist, die substituiert oder unsubstituiert ist; und
- Sp" eine aliphatische lineare, verzweigte oder cyclische Kette mit zumindest zwei Kohlenstoffatomen ist,
- Y aus -O-, -C(=O)O, -OC(=O)- ausgewählt ist,
- oder der allgemeinen Formel: 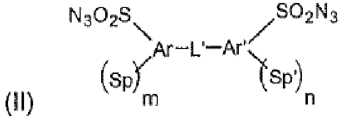
wobei:
- Ar und Ar' aromatische Bausteine sind;
- L' -O- ist;
- m 1 ist und n 0 ist; oder m 0 ist und n 1 ist;
Sp und Sp' organische Ketten mit zumindest zwei Kohlenstoffatomen sind, wobei Sp und/oder Sp' eine aliphatische lineare, verzweigte oder cyclische Kette ist, die zumindest drei Kohlenstoffatome umfasst,
als Vernetzungsmittel.

9. Verwendung nach Anspruch 8 der Verbindung der allgemeinen Formel (l) oder (II) nach einem der Ansprüche 1 bis 6 oder der Zusammensetzung nach Anspruch 7.

10. Verwendung nach einem der Ansprüche 8 bis 9 als Vernetzungsmittel bei der Herstellung von Polymeren.

11. Verwendung nach einem der Ansprüche 8 bis 10 als Vernetzungsmittel bei der Herstellung von Polyolefinen, Polyolefincopolymeren und Mischungen von Polyolefinen, vorzugsweise bei der Herstellung von Polypropylen oder Polyethylen.

12. Verwendung nach einem der Ansprüche 8 bis 11 als Vernetzungsmittel bei der Herstellung von Elastomeren, Elastomercopolymeren und Mischungen von Elastomeren, vorzugsweise bei der Herstellung von EPM, EPDM und E PM-Polyolefinmischungen.

## Revendications

1. Composé de formule générale
(I) (N₃O₂S-Ar-Y) ₂Sp",
dans laquelle
- Ar est un groupe phényle substitué ou non substitué ; et
- Sp" est une chaîne aliphatique linéaire, ramifiée ou cyclique ayant au moins deux atomes de carbone,
- Y est choisi parmi -O-, -C(=O)O, -OC(=O)-.

2. Composé selon la revendication 1, dans lequel Y est -O- ; et la chaîne organique Sp" est une chaîne aliphatique linéaire, ramifiée ou cyclique qui comprend de préférence au moins 3, de préférence au moins 4, mieux encore au moins 5 et tout spécialement de 6 à 16 atomes de carbone.

3. Composé selon l'une quelconque des revendications 1 et 2, qui est :
- le composé 1,6-bis(4-azidosulfonylphénoxy)hexane ou le composé 1,10-bis(4-azidosulfonylphénoxy)décane ou
- le composé 1,2-bis(4-azidosulfonylphénoxy)hexadécane ou
- un mélange d'isomères de 4,8-bis(4-azidosulfonylphénoxyméthyl)tricyclo[5.2.1.0^{2,6}]décane, ou
- le composé 1,4-bis(4-azidosulfonylphénoxyméthyl-cyclohexane ou
- le composé éther de 1,n-bis(4-azidosulfonylphényl)-polyéthylèneglycol, ou
- le 1,6-bis(4-azidosulfonyl-3-pentadécylphénoxy)hexane.

4. Composé de formule générale 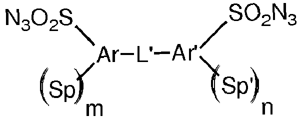 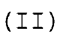 dans laquelle :
- Ar et Ar' sont des groupes phényle ;
- L' est -O- ;
- m vaut 1 et n vaut 0 ; ou m vaut 0 et n vaut 1 ;
- Sp et Sp' sont des chaînes organiques ayant au moins deux atomes de carbone,
dans lequel Sp et/ou Sp' sont des chaînes aliphatiques linéaires, ramifiées ou cycliques comprenant au moins 3 atomes de carbone.

5. Composé selon la revendication 4, dans lequel Sp et/ou Sp' sont des chaînes aliphatiques linéaires, ramifiées ou cycliques comprenant au moins 4, mieux encore au moins 5 et tout spécialement de 6 à 16 atomes de carbone.

6. Composé selon l'une quelconque des revendications 4 et 5, qui est
- le composé 4,4'-bis(azidosulfonyl)-3-pentadécyl-diphényléther ou
- le composé 4,4'-bis(azidosulfonyl)-2-hexadécyl-diphényléther et/ou le 4,4'-bis(azidosulfonyl)-2-octadécyl-diphényléther.

7. Composition comprenant le composé de l'une quelconque des revendications 1 à 6, et comprenant au plus 50 % (p/p), de préférence au plus 40 % (p/p), mieux encore au plus 30 % (p/p), plus particulièrement au plus 20 % (p/p), tout spécialement au plus 10 % (p/p) d'un agent de phlegmatisation, ou qui est sans agent de phlegmatisation.

8. Utilisation du composé de formule générale (I) (N₃O₂S-Ar-Y) ₂Sp", dans laquelle
- Ar est un groupe phényle substitué ou non substitué ; et
- Sp" est une chaîne aliphatique linéaire, ramifiée ou cyclique ayant au moins deux atomes de carbone,
- Y est choisi parmi -O-, -C(=O)O, -OC(=O)-,
ou de formule générale : 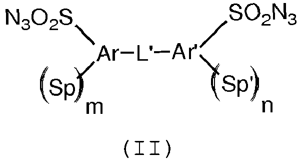 dans laquelle :
- Ar et Ar' sont des blocs de construction aromatiques ;
- L' est -O- ;
- m vaut 1 et n vaut 0 ; ou m vaut 0 et n vaut 1 ;
- Sp et Sp' sont des chaînes organiques ayant au moins deux atomes de carbone, lesquels Sp et/ou Sp' sont des chaînes aliphatiques linéaires, ramifiées ou cycliques comprenant au moins 3 atomes de carbone,
en tant qu'agent de réticulation.

9. Utilisation selon la revendication 8 du composé de formule générale (I) ou (II) tel que défini dans l'une quelconque des revendications 1 à 6, ou de la composition telle que définie dans la revendication 7.

10. Utilisation selon l'une quelconque des revendications 8 et 9, en tant qu'agent de réticulation dans la fabrication de polymères.

11. Utilisation selon l'une quelconque des revendications 8 à 10, en tant qu'agent de réticulation dans la fabrication de polyoléfines, de copolymères de polyoléfine et de mélanges de polyoléfines, de préférence dans la fabrication de polypropylène ou de polyéthylène.

12. Utilisation selon l'une quelconque des revendications 8 à 11, en tant qu'agent de réticulation dans la fabrication d'élastomères, de copolymères d'élastomère et de mélanges d'élastomères, de préférence dans la fabrication d'EPM, d'EPDM et de mélanges EPM-polyoléfine.
